# EUROPÄISCHE PATENTSCHRIFT

(11) **EP 2 602 004 B1**
(45) Veröffentlichungstag und Bekanntmachung des Hinweises auf die Patenterteilung: **09.12.2015**
(21) Anmeldenummer: 12189016.4
(22) Anmeldetag: 18.10.2012
(51) Int. Cl.: A61N 5/10

(54) **Bestrahlungsplanungverfahren und Bestrahlungsplanungsvorrichtung für die Partikeltherapie**
Irradiation planning method and irradiation planning device for particle therapy
Procédé de planification d'irradiation et dispositif de planification d'irradiation pour la thérapie à particules

(30) Priorität: 09.12.2011 DE 102011088160
(43) Veröffentlichungstag der Anmeldung: 12.06.2013
(73) Patentinhaber: Siemens Aktiengesellschaft, 80333 München (DE)
(72) Erfinder: Chen, Wenjing, 69221 Dossenheim (DE); Gemmel, Alexander, 91054 Erlangen (DE); Rietzel, Eike, 64331 Weiterstadt (DE)

(56) Entgegenhaltungen:
- EP-A1- 2 108 402
- US-A1- 2011 272 600
- US-B2- 7 268 358
- KRAEMER M ET AL: "TREATMENT PLANNING FOR HEAVY-IRON RADIOTHERAPY: PHYSICAL BEAM MODEL AND DOSE OPTIMIZATION", PHYSICS IN MEDICINE AND BIOLOGY, INSTITUTE OF PHYSICS PUBLISHING, BRISTOL GB, Bd. 45, Nr. 11, 1. November 2000 (2000-11-01), Seiten 3299-3317, XP001146537, ISSN: 0031-9155, DOI: 10.1088/0031-9155/45/11/313

## Beschreibung

Die Erfindung betrifft ein Verfahren zur Bestrahlungsplanung für ein Zielvolumen für die Partikeltherapie, insbesondere bei Bestrahlungsplanungen, bei denen die zu applizierende Partikelzahl zielpunktweise im Zielvolumen festgelegt wird. Ebenso betrifft die Erfindung eine entsprechende Bestrahlungsplanungsvorrichtung.

Im Vorfeld einer Partikeltherapie-Behandlung ist es notwendig, einen Bestrahlungsplan zu bestimmen. Im Wesentlichen wird durch den Bestrahlungsplan festgelegt, wie das Zielvolumen zu bestrahlen ist, um eine gewünschte Dosisverteilung im Körper zu applizieren. Im Falle der Partikeltherapie wird oftmals Zielpunkten oder Rasterpunkten des Zielvolumens jeweils die dort zu applizierende Teilchenzahl festgelegt.

Zur Bestrahlungsplanung wird hierzu üblicherweise ein iteratives Optimierungsverfahren eingesetzt. Aufgrund der Komplexität der Berechnung dauert es mitunter lange und erfordert einen hohen Rechenaufwand, einen Bestrahlungsplan derart zu bestimmen, dass bestimmte gewünschte Dosisvorgaben hinsichtlich einer Bestrahlung des Zielvolumen und der umliegenden Organe erfüllt sind.

Diese Bestimmung eines adäquaten Bestrahlungsplans ist zudem deshalb verkompliziert, da sich die interne Anatomie eines Patienten mit der Zeit verändern kann. Beispielsweise können Zielvolumina innerhalb des Abdomens ihre Lage von Tag zu Tag ändern oder im Verlauf mehrerer Tage oder Wochen. Ein typisches Organ, das oftmals einer Positionsänderung unterliegt, ist die Prostata. So können z.B. die neben der Prostata gelegene Blase und das neben der Prostata gelegene Rektum einen Einfluss auf die Lage und die Form der Prostata haben, je nach Füllungszustand der Blase und/oder des Rektums.

Daher kann ein Bestrahlungsplan, der an einem Tag berechnet worden ist, nicht mehr optimal sein, um den Patienten an einem anderen Tag zu bestrahlen.

Es die Aufgabe der Erfindung, ein Verfahren zur Bestrahlungsplanung bereitzustellen, das eine schnelle Bestrahlungsplanung ermöglicht und eine genaue Anpassung des Bestrahlungsplans an das Zielvolumen erlaubt. Weiterhin ist es die Aufgabe der Erfindung, eine entsprechende Bestrahlungsplanungsvorrichtung anzugeben.

Die Aufgabe der Erfindung wird durch die Gegenstände der unabhängigen Ansprüche gelöst.

Das erfindungsgemäße Verfahren zur Erstellung eines Bestrahlungsplans für ein Zielvolumen umfasst folgende Schritte:
- Bereitstellen einer Modulationskurve, welche die Modulation von Partikelzahlen bei Zielpunkten, welche im Zielvolumen in Strahlrichtung hintereinander liegend, charakterisiert,
- Ermitteln einer aktuellen Lage des Zielvolumens,
- Bestimmen einer Vielzahl von aktuellen Zielpunkten, welche das Zielvolumen in seiner aktuellen Lage überdeckenden,
- Zuordnen von Partikelzahlen zu den aktuellen Zielpunkten unter Verwendung der Modulationskurve.

Die Erfindung beruht auf der Erkenntnis, dass ein iteratives Optimierungsverfahren, das üblicherweise zur Bestrahlungsplanung eingesetzt wird, lange dauert und rechenaufwändig ist.

Stattdessen wird nun vorgeschlagen, zur Erreichung eines vorgegebenen Planungsziels die Bestrahlungsplanung mithilfe einer Modulationskurve durchzuführen.

Es wurde erkannt, dass bei einem bereits erstellten Bestrahlungsplan die Modulation der Partikelzahlen für hintereinander liegende Zielpunkte durch das gesamte Zielvolumen hinweg nur eine geringe Variation aufweist. Die Richtungsangabe "hintereinander" bezieht sich hier auf die Strahlrichtung. Hintereinander liegende Zielpunkte sind folglich in Strahlrichtung des Partikelstrahls hintereinander angeordnet. Hintereinander liegende Zielpunkten können unterschiedliche Energieschichten zugeordnet sein. Ein weiter distal liegender Zielpunkt hat dabei ein höheres Energieniveau als ein weiter proximal liegender Zielpunkt.

Darüber hinaus ist die Modulation der Partikelzahlen in Strahlrichtung gesehen auch vergleichsweise ähnlich zwischen Bestrahlungsplänen, die für unterschiedliche CT-Datensätze des gleichen Patienten berechnet wurden; die Modulation ist sogar zwischen verschiedenen Patienten vergleichbar.

Auf dieser Analyse beruht nun die Erkenntnis, dass zur Bestrahlungsplanung nun nicht mehr das aufwändige Optimierungsverfahren eingesetzt werden muss, sondern dass die Bestrahlungsplanung anhand einer Modulationskurve durchgeführt werden kann.

Die Modulationskurve beschreibt, wie sich die Partikelzahlen eines Bestrahlungsplans für das zu bestrahlende Zielvolumen in Strahlrichtung gesehen innerhalb des Zielvolumens verändern. In den allermeisten Fällen beschreibt die Modulationskurve den Abfall der Partikelzahlen von distal nach proximal. Implementiert werden kann die Modulationskurve beispielsweise durch eine analytische Funktion, oder aber auch durch ein z.B. eindimensionales Array, aus dem entnommen werden kann, wie sich die Partikelzahlen bei hintereinander liegenden Zielpunkten ändern.

Da sich die Modulationskurve auf die Modulation der Partikelzahlen in Strahlrichtung bezieht, kann die Modulationskurve auch als Tiefenmodulationskurve bezeichnet werden (engl.: "depth modulation curve" - DMC).

Ein Aspekt der Erfindung ist es nun, die Modulationskurve bzw. Tiefenmodulationskurve aus den Partikelzahlen eines bereits bestehenden Plans (oder gar aus mehreren bestehenden Plänen) zu extrahieren. Diese Modulationskurve kann dann als Basis für die Bestimmung eines neuen Plans verwendet werden, genauer für die Bestimmung der Partikelzahlen bei einem neuen Plan.

Im Vergleich zu einer herkömmlichen Optimierung, die auf einer iterativen Optimierung einer Zielfunktion basiert, um Dosisvorgaben hinsichtlich einer gewünschten Dosisverteilung zu erreichen, stellt das vorgeschlagene Verfahren zwar nur eine Näherungsmethode dar, die aber in vielen Fällen ausreichend gute Ergebnisse liefert. Mithilfe dieser Näherungsmethode ist eine schnelle Planadaptation möglich.

Eine Anpassung einer existierenden Bestrahlungsplanung (engl: "Re-Planning") kann auf diese Weise sehr schnell, zum Beispiel innerhalb 1 min, erledigt werden, wenn die aktuelle Lage des Zielvolumens - z.B. die aktuelle Kontur des Zielvolumens - zur Verfügung steht. Das vorgeschlagene Verfahren zur Planadaption kann sowohl bei einer Strahlanpassung online geschehen, z.B. während einer Bestrahlungsfraktion oder gar während der Strahlapplikation, als auch beim offline Re-Planning eingesetzt werden. Es ist sogar denkbar, dass Verfahren für eine Planung bei einem neuen Patienten einzusetzen, für den noch keine Bestrahlungsplanung durchgeführt wurde.

Die das Zielvolumen überdeckenden Zielpunkte, für die dann Partikelzahlen ermittelt und zugeordnet worden sind, können beispielweise in einem Scanverfahren bestrahlt werden, indem ein Partikelstrahl sukzessive nacheinander auf jeweils einen Zielpunkt gerichtet wird, bis an den Zielpunkten jeweils die zugeordnete Teilchenzahl appliziert worden ist. Als Scanverfahren können z.B. Rasterscan-Verfahren eingesetzt werden, bei denen der Partikelstrahl von Rasterpunkt zu Rasterpunkt scannt. Die Zielpunkte können auch als Rasterpunkte bezeichnet werden. Es sind aber auch andere Scanverfahren möglich, wie z.B. ein kontinuierliches Scanning. Ebenfalls können bei dem Scanning auch verschiedene Varianten angewendet werden, wie z.B. ein Rescanning, bei dem die Rasterpunkte mehrfach zur Dosisapplikation angefahren werden.

Insbesondere kann die Modulationskurve in einem Vorverfahren aus mindestens einem bestehenden Bestrahlungsplan, in welchem eine Zuordnung von Partikelzahlen zu Zielpunkten des Zielvolumens hinterlegt ist, ermittelt werden.

Der bestehende Bestrahlungsplan kann beispielsweise das gleiche Bestrahlungsziel - also die gleichen Vorgaben hinsichtlich Dosis für das Zielvolumen, Dosis für Risikoorgane etc. - aufweisen wie der Bestrahlungsplan, der durch das erfindungsgemäße Verfahren ermittelt werden soll. Falls mehrere bestehende Bestrahlungspläne verwendet werden, kann z.B. aus jedem Bestrahlungsplan eine Modulationskurve ermittelt werden und anschließend - ggf. nach Normierung - gemittelt werden.

Es ist dabei möglich, dass das Zielvolumen, das in dem Vorverfahren zur Ermittlung der Modulationskurve verwendet wird, zu derselben Person gehört wie das Zielvolumen, für das die aktuelle Lage ermittelt wird. Auf diese Weise findet ein Re-Planning für den gleichen Patienten statt kann beispielsweise dafür eingesetzt werden, einen bestehenden Bestrahlungsplan an eine aufgetretene Lage- und/oder Formveränderung des Zielvolumens anzupassen.

Es ist aber auch möglich, dass das Zielvolumen, das in dem Vorverfahren zur Ermittlung der Modulationskurve verwendet wird, zu einer anderen Person gehört als das Zielvolumen, für das die aktuelle Lage ermittelt wird. Beispielsweise kann ein bestehender Bestrahlungsplan verwendet werden, der für das Zielvolumen bei einer Person erstellt worden ist, um hieraus die Modulationskurve zu ermitteln, die dann in dem erfindungsgemäßen Verfahren für die Erstellung eines Bestrahlungsplans für das gleiche Zielvolumen bei einer anderen Person eingesetzt wird.

Es ist selbst möglich, dass das Zielvolumen, das in dem Vorverfahren zur Ermittlung der Modulationskurve verwendet wird, zu einem Phantom gehört, welches das Zielvolumen nachbildet. Auch ein Bestrahlungsplan für ein in einem Phantom nachgebildetes Zielvolumen kann bereits ausreichen, eine Modulationskurve zu ermitteln, die dann zur Erstellung von Bestrahlungsplänen von Personen eingesetzt wird.

Die Modulationskurve kann bei dem Vorverfahren ermittelt werden, indem Partikelzahlen mehrerer Zielpunktlinien gemittelt werden. Eine Zielpunktlinie ist dabei durch in Strahlrichtung hintereinander liegende Zielpunkte gegeben. Eine Zielpunktlinie beschreibt den Weg eines Nadelstrahls durch das Zielvolumen, bzw. die Zielpunkte, die der Nadelstrahl hierbei trifft. Dies kann eine Reihe von Zielpunkten sein, die in Strahlrichtung hintereinander liegen.

Für jede Zielpunktlinie der mehreren Zielpunktlinien kann der Abfall der Partikelzahlen von distal nach proximal ermittelt werden. Anschließend können die den unterschiedlichen Zielpunktlinien zugeordneten Partikelzahlmodulationen gemittelt und hieraus die Modulationskurve erstellt werden.

Die Modulationskurve kann hinsichtlich des für die Bestrahlung des Zielvolumens notwendigen Energieniveaus des Partikelstrahls normiert sein. Die Normierung bedeutet letztlich, dass die Partikelzahlen mit einem Faktor multipliziert werden. Die Normierung kann beispielsweise dadurch erfolgen, dass durch die Partikelzahlen durch das Energieniveau der distalsten Energieschicht dividiert werden. Diese Normierung ist insbesondere dann hilfreich, wenn die Modulationskurve für die Ermittlung von Partikelzahlen für ein Zielvolumen, das zur Bestrahlung ein zum Referenzzielvolumen unterschiedliches Energieniveau des Partikelstrahls erfordert, verwendet werden soll, wenn also die zur Bestrahlung erforderliche Reichweite des Partikelstrahls eine Anpassung erfordert. So kann z.B. der Bestrahlungsplan, aus dem die Modulationskurve ermittelt wird, eine andere (maximale) Reichweite des Partikelstrahls erfordern als das Zielvolumen, für dessen aktuelle Lage der Bestrahlungsplan anhand der Modulationskurve ermittelt wird.

Ungenauigkeiten, die sich aufgrund des Transfers der Partikelzahlen von einem bestehenden Plan auf einen neuen Plan ergeben würden, falls eine allzu sehr abweichende Reichweite des Partikelstrahls zwischen dem neuen und dem bestehenden Plan vorhanden ist, werden durch die Normierung ausgeglichen. Beim Transfer von einem bestehenden auf einen neuen Plan können die Partikelzahlen insgesamt mit einem Faktor multipliziert werden, der dem Quotienten der maximale Energie des Partikelstrahl bei dem neuen Plan und der maximale Energie des Partikelstrahl bei dem alten Plan entspricht.

In einer Ausgestaltung des Verfahrens wird die aktuelle Lage des Zielvolumens bestimmt, indem eine Registrierung eines aktuellen dreidimensionalen Bilddatensatzes, z.B. eines CT-Datensatzes, zu einem früheren dreidimensionalen Datensatz, beispielsweise einem Planungs-CT, durchgeführt wird, wobei die Form und/oder die Lage des Zielvolumens in dem früheren Datensatz bekannt ist. Die Form und/oder die Lage des Zielvolumens aus dem früheren Datensatz kann so auf den aktuellen Datensatz übertragen werden. Beispielsweise kann eine Kontur des Zielvolumens von einem Datensatz auf einen weiteren Datensatz übertragen werden, um die aktuelle Form und/oder Lage des Zielvolumens zu kennzeichnen.

Beim Bestimmen der Vielzahl von Zielpunkten, die das Zielvolumen in seiner aktuellen Lage überdecken, kann ein Zielpunktabstand, z.B. in Strahlrichtung oder in einer Richtung senkrecht zur Strahlrichtung, gewählt werden, der einem bereits bestehenden Referenzplan entspricht.

Beispielsweise kann der Zielpunktabstand desjenigen Referenzplans übernommen werden, aus dem die Modulationskurve ermittelt wurde. Auf diese Weise lassen sich Partikelzahlen besonders leicht aus der Modulationskurve auf die aktuellen Zielpunkte übertragen. Z.B. kann eine Interpolation von die Modulationskurve beschreibenden Arrayelementen damit vermieden werden.

In einer Ausführungsform des Verfahrens kann beim Zuordnen von Partikelzahlen zu den aktuellen Zielpunkten einer Zielpunktlinie, die aus aktuellen Zielpunkten gebildet ist, diejenigen Partikelzahlen zugeordnet werden, die durch die Modulationskurve beschrieben werden.

Partikelzahlen, die durch die Modulationskurve beschrieben werden und die den aktuellen Zielpunkten zugeordnet werden, können vor Zuordnung zu einer Zielpunktlinie aus aktuellen Zielpunkten in Bezug auf das Energieniveau, das für die Bestrahlung des Zielvolumens notwendig ist, angepasst werden. Auf diese Weise kann das Verfahren unterschiedliche Eindringtiefen im Vergleich zum Referenzzielvolumen, die durch einer Lage- und/oder Formveränderung des aktuellen Zielvolumens bedingt sind, ausgleichen.

Das Verfahren kann zur Berechnung eines Bestrahlungsplans mit optimierter physikalischer oder biologischer Dosisverteilung eingesetzt werden.

Insbesondere bei einem Bestrahlungsplan mit biologisch optimierter Dosisverteilung ist das vorgeschlagene Verfahren vorteilhaft, da es im Vergleich zur herkömmlichen Optimierung mit einer iterativ zu optimierenden Zielfunktion eine besonders große Zeitersparnis ermöglicht.

Das Verfahren kann zur Übertragung eines bereits bestehenden Bestrahlungsplans auf einen neuen Bestrahlungsplan eingesetzt werden. Der bestehende Bestrahlungsplan und der neue Bestrahlungsplan können dabei dem gleichen Patienten zugeordnet sein. Der bestehende Bestrahlungsplan und der neue Bestrahlungsplan können aber selbst unterschiedlichen Patienten zugeordnet sein. Es ist selbst möglich, dass der bestehende Bestrahlungsplan einem Phantom und der neue Bestrahlungsplan einem Patienten zugeordnet ist.

Es ist auch möglich, dass das Verfahren zur Bestimmung von Partikelzahlen bei einem Bestrahlungsfeld durchgeführt wird, wobei die Modulationskurve nicht aus dem ersten, sondern aus einem zweiten oder dritten Bestrahlungsfeld ermittelt wird, das im Vergleich zum ersten Bestrahlungsfeld einen unterschiedlichen Einstrahlwinkel aufweist.

Diese große Flexibilität des Verfahrens ist möglich, da erkannt wurde, dass eine Modulationskurve eine große Ähnlichkeit besitzt, selbst zwischen verschiedenen Personen, zwischen Phantomen und Personen und auch über verschiedene Bestrahlungsfelder hinweg.

In einer Ausführungsform des Verfahrens können mehrere Modulationskurven bereitgestellt werden, welche z.B. in einer Datenbank hinterlegt werden können. Diese Modulationskurven können aus unterschiedlichen Bestrahlungsplänen erzeugt worden sein, die sich hinsichtlich Zielorgan, Zieldosis, zu verwendende Teilchensorte und/oder Zielpunktabständen unterscheiden. Bei der Zuordnung von Partikelzahlen zu den aktuellen Zielpunkten kann nun eine der Modulationskurven ausgewählt werden, insbesondere diejenige, bei denen die Vorgaben des ursprüngliche Bestrahlungsplans am besten mit den Vorgaben des neuen Bestrahlungsplans übereinstimmen.

Die erfindungsgemäße Bestrahlungsplanungsvorrichtung hat eine Rechnervorrichtung und umfasst:
eine Komponente zum Bereitstellen einer Modulationskurve, welche die Modulation von Partikelzahlen bei Zielpunkten, welche im Zielvolumen in Strahlrichtung hintereinander liegend, charakterisiert,
eine Komponente zum Ermitteln einer aktuellen Lage des Zielvolumens,
eine Komponenten zum Bestimmen einer Vielzahl von aktuellen Zielpunkten, welche das Zielvolumen in seiner aktuellen Lage überdeckenden,
eine Komponente zum Zuordnen von Partikelzahlen zu den aktuellen Zielpunkten unter Verwendung der Modulationskurve.

Die Rechnervorrichtung kann insbesondere derart ausgebildet sein, dass eine Ausgestaltung des Verfahrens wie oben beschrieben implementiert wird.

Die vorangehende und die folgende Beschreibung der einzelnen Merkmale, deren Vorteile und deren Wirkungen bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Ausführungsformen der Erfindung werden anhand der folgenden Zeichnung näher erläutert, ohne jedoch darauf beschränkt zu sein. Es zeigen:
- Fig. 1: ein Ablaufdiagramm für eine Ausführungsform des Verfahrens, mit dessen Hilfe ein Bestrahlungsplan aus einer Partikelzahlenkurve berechnet wird,
- Fig. 2: eine Darstellung von Isoenergieschichten, Rasterpunktschichten und Strahllinien,
- Fig. 3: und Fig. 4 eine schematische Darstellung von Rasterpunkten und Isoenergieschichten zur Erläuterung von Verfahrensschritten,
- Fig. 5: unnormierte Modulationskurven, die aus physikalischen Behandlungsplänen abgeleitet wurden,
- Fig. 6: und Fig. 7 normierte Modulationskurven für verschiedene physikalische bzw. biologische Bestrahlungspläne,
- Fig. 8 bis Fig. 11: die Ergebnisse einer Analyse der Güte des Verfahrens bei einer Intra-Patienten-Adaptation,
- Fig. 12 bis Fig. 15: die Ergebnisse einer Analyse der Güte des Verfahrens bei einer Inter-Patienten-Adaptation,
- Fig. 16 bis Fig. 18: die Datensätze von verschiedenen Phantomen für eine Prostata-Bestrahlung, und
- Fig. 19 bis Fig. 22: die Ergebnisse einer Analyse der Güte des Verfahrens bei einer Phantom-Patienten-Adaptation und Interfeld-Adaptation.

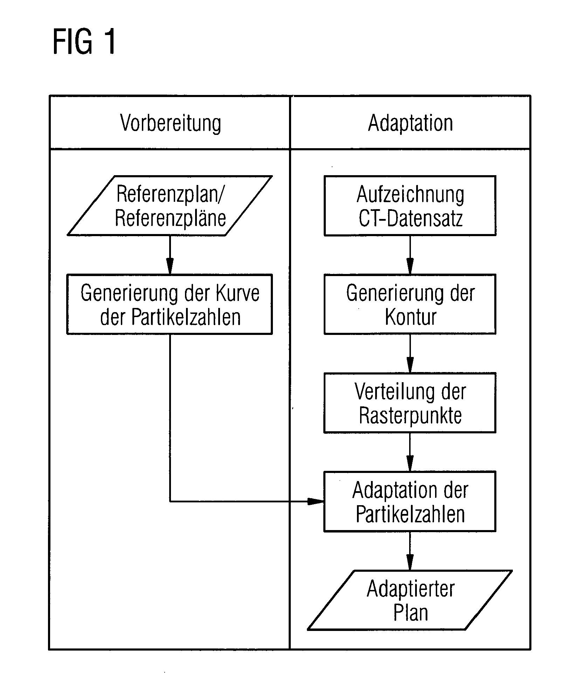

Zunächst wird der Ablauf des Verfahrens anhand von Fig. 1 bis Fig. 4 dargestellt.

Fig. 1 zeigt die Verfahrensschritte für den Ablauf des vorgeschlagenen Adaptationsverfahrens.

Für die Vorbereitung des Verfahrens wird eine Partikelzahlenkurve generiert, die die Modulation der Partikelzahlen in unterschiedlicher Tiefe im Zielvolumen beschreibt, daher auch Tiefenmodulationskurve (engl.: "depth modulation curve", DMC) genannt. Die DMC wird aus einem oder mehreren Referenzplänen erzeugt.

Dies kann beispielsweise der ursprüngliche Patientenplan sein z.B. für eine Online-Adaptation der Bestrahlungsplanung oder für ein sogenanntes Re-Planning. Es kann aber auch der Plan eines Referenzpatienten oder ein Plan für ein Phantom sein. Aus diesem Plan (oder aus mehreren Plänen) kann aber auch eine Bestrahlungsplanung für einen neuen Patienten "von Grund auf" neuberechnet werden.

Zur Berechnung eines Plans an eine aktuelle Patientenanatomie wird ein CT-Datensatz aufgezeichnet. Das Zielvolumen bzw. dessen Kontur wird eingezeichnet, z.B. durch einen Anwender oder durch eine deformierbare Transformation eines bereits vorliegenden Referenz-Zielvolumens. Das Isozentrum des Zielvolumens wird definiert.

Rasterpunkte werden über das neue Zielvolumen verteilt, und Partikelzahlen werden für jeden Rasterpunkt aus der Modulationskurve erzeugt.

Auf diese Weise erhält man einen an die aktuelle Patientenanatomie angepassten Bestrahlungsplan.

Im Folgenden wird das Verfahren zur Erzeugung der DMC, zur Verteilung der Rasterpunkte und zur Adaptation der Partikelzahlen näher erläutert.

### Generierung der DMC

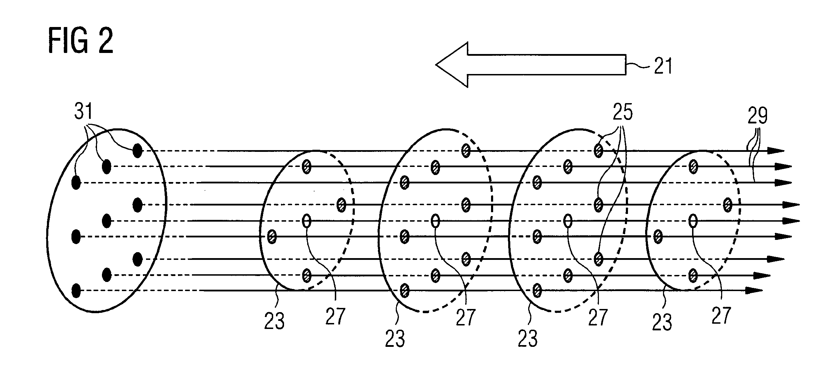

Zur Illustration wird in Fig. 2 ein vereinfachtes, aus vier Isoenergieschichten 23 aufgebautes, stark schematisiertes, konvexes Zielvolumen gezeigt, das mit einer Vielzahl von Rasterpunkten 25 überdeckt ist.

Der dicke Pfeil 21 zeigt die Einstrahlrichtung des Partikelstrahls. Die distale Schicht befindet sich links, die proximale Schicht befindet sich rechts. In jeder Schicht stellt der zentrale Rasterpunkt 27 das Isozentrum dar.

Die Rasterpunkte 25 können entlang einer Strahllinie 29 (d.h. in Strahlrichtung) projiziert werden. Bei dem hier dargestellten Zielvolumen ergeben sich so insgesamt neun Strahllinien 29 bzw. neun Rasterpunktprojektionen 33.

Details zur Bestrahlung eines Zielvolumens, wie z.B. die Rasterpunktpositionen, die Energieniveaus und die Feldinformationen (z.B. die Anzahl der Felder und die Einfallswinkel des Strahls) können aus einem oder mehreren Referenzplänen entnommen werden. Der bzw. die Referenzpläne wurden mit einem herkömmlichen Bestrahlungsplanungsverfahren erstellt und hinsichtlich der vorgeschriebenen physikalischen Dosis oder RBEgewichteten Dosis optimiert.

Die Modulationskurve DMC wird nun für jedes Feld des Bestrahlungsplans bzw. der Bestrahlungspläne erzeugt, indem folgende Schritte durchgeführt werden:
In einem ersten Schritt werden bei einem Referenzplan die Rasterpunkte auf eine Ebene senkrecht zur Strahlrichtung wie in Fig. 2 illustriert projiziert.

Anschließend werden für jede Strahllinie, welche durch Rasterpunkte mit gleicher Rasterpunktprojektion geht, die Partikelzahlen für jede Energieschicht entlang der Strahllinie extrahiert. Diese können in einem eindimensionalen Array oder Vektor hinterlegt werden, mit dem am weitesten distal gelegenen Rasterpunkt als Startpunkt des Vektors.

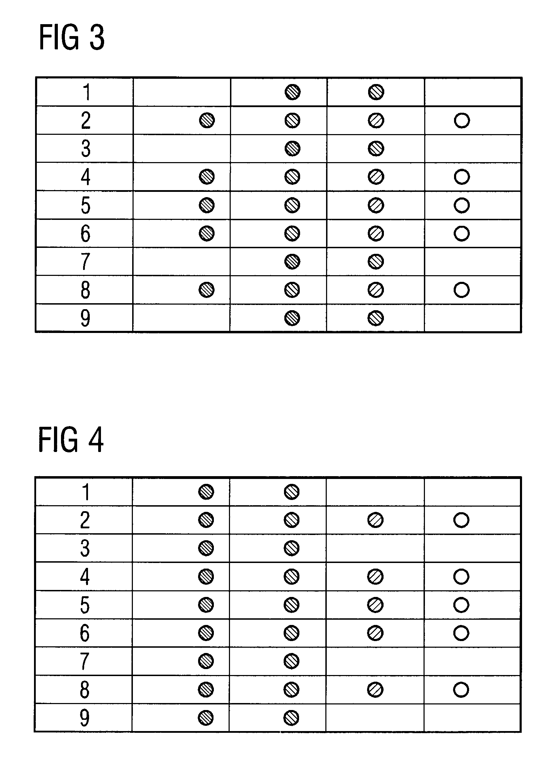

Fig. 3 zeigt die zu den neun Strahllinien gehörigen Rasterpunkte bzw. Partikelzahlvektoren in tabellarischer Form. Die linke Spalte entspricht der distalen Isoenergieschicht, die rechte Spalte der proximalen Isoenergieschicht. Die Schraffurdichte kennzeichnet qualitativ die den Rasterpunkten zugeordnete Partikelzahl. Man sieht einen Abfall der Partikelzahlen von distal nach proximal.

Anschließend werden alle Partikelzahlvektoren derart verschoben, dass die Startwerte alle bei der Energieschicht der distalen Kante beginnen.

Fig. 4 zeigt die zu den neun Strahllinien gehörigen, auf die distale Isoenergieschicht geschobenen Partikelzahlvektoren in tabellarischer Form. Die Vektoren beginnen nun bei der distalsten Isoenergieschicht, also bei der Schicht, bei der der Partikelstrahl wieder aus dem Zielvolumen austreten würde.

Nun kann die Tiefenmodulationskurve berechnet werden, indem der Mittelwert der verschobenen Partikelzahlenvektoren berechnet wird. Im Prinzip wird über die Spalten aus Fig. 4 gemittelt.

Anschließend werden die Partikelzahlen des gemittelten Vektors (also die Partikelzahlen entlang der DMC) durch das Energieniveau der am weitesten distal gelegenen Energieschicht dividiert. Durch diese Normierung können Unterschiede hinsichtlich der Lage des Zielvolumens im zu bestrahlenden Patienten/Objekt ausgeglichen werden.

Falls mehrere Referenzpläne vorhanden sind, können die normierten DMCs zu jedem dieser Pläne ermittelt werden und anschließend gemittelt werden.

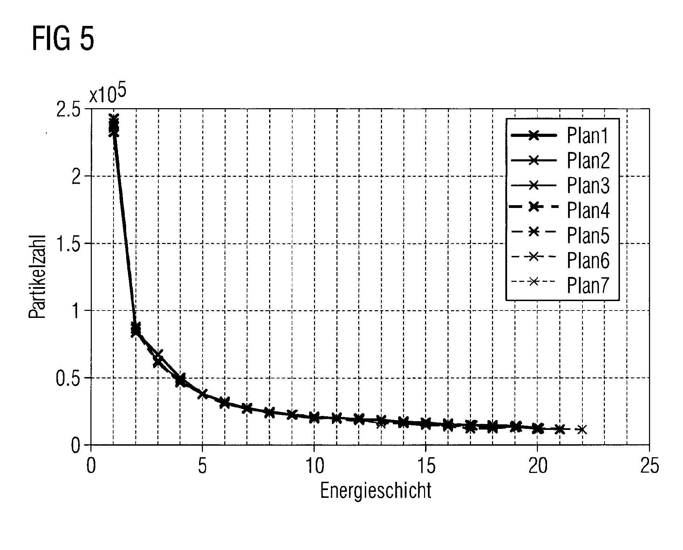

Fig. 5 zeigt die unnormierten DMCs (engl.: "particle number" für Partikelzahl gegen engl. "energy slice" für Energieschicht), die aus physikalischen Behandlungsplänen abgeleitet wurden, die aus verschiedenen CT-Datensätzen des gleichen Prostata-Patienten erzeugt wurden und die ein Bestrahlungsfeld (F1) betreffen. Zieldosis war eine physikalische Dosis von 1 Gy bei zwei einander gegenüberliegenden Feldern (+90° und -90°). Die unnormierten DMCs stimmen sehr gut überein.

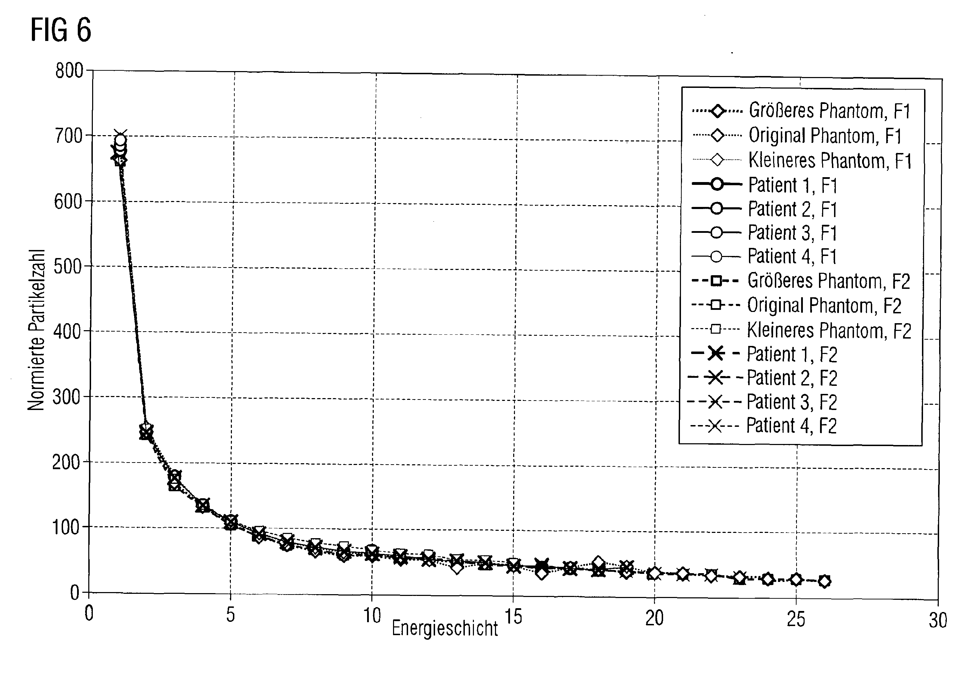

Fig. 6 zeigt normierte DMCs ("normalized particle number" für normierte Partikelzahl gegen "energy slice" für Energieschicht) für verschiedene Bestrahlungspläne, die entweder zu Phantomen (engl.: "larger phantom", "original phantom", "smaller phantom") oder zu unterschiedlichen Patienten ("patient 1" bis "patient 4") gehören, für verschiede Bestrahlungsfelder (F1 und F2). Die Bestrahlungspläne sind dabei Bestrahlungspläne, die hinsichtlich der physikalischen Dosis optimiert wurden.

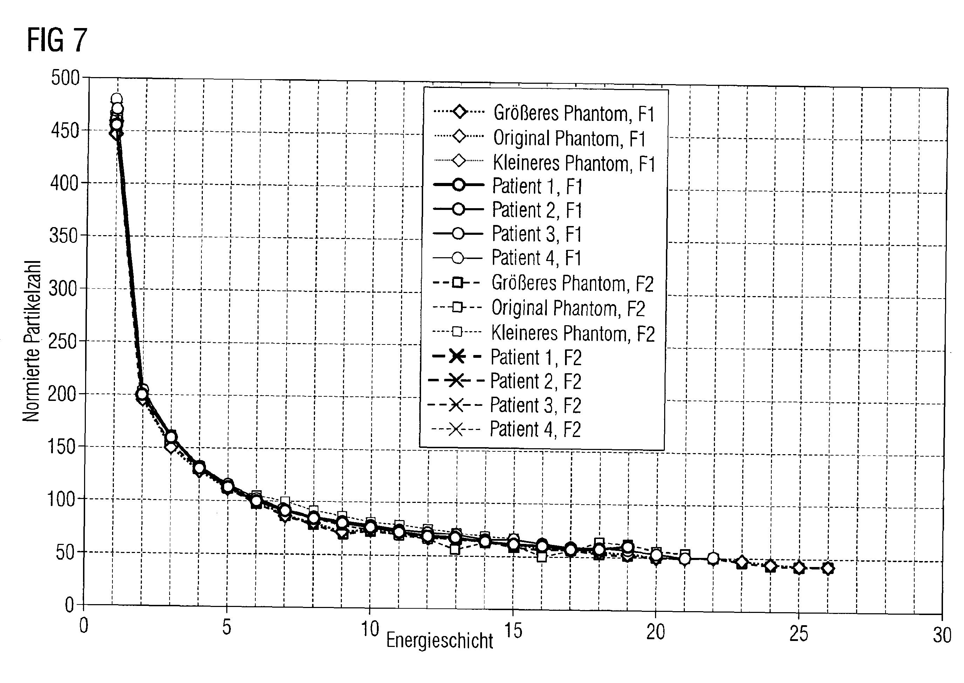

Fig. 7 zeigt eine Darstellung von Partikelzahlenkurven analog zu Fig. 6, nur dass die DMCs diesmal aus Bestrahlungsplänen, die hinsichtlich der biologischen Dosis optimiert wurden, erstellt wurden.

Den physikalischen Bestrahlungsplänen bei Fig. 6 lag eine Fraktionsdosis von jeweils 1 Gy zu Grunde. Den biologischen Bestrahlungsplänen bei Fig. 7 lag eine Fraktionsdosis von jeweils 3 Gy zu Grunde. Auch hier waren die Bestrahlungspläne für jeweils zwei gegenüberliegende Bestrahlungsfelder optimiert.

Zu sehen ist, dass die DMCs der biologischen Pläne etwas stärker voneinander differieren als die DMCs der physikalischen Pläne. Dennoch stimmten alle DMCs weiterhin gut überein.

### Bestimmung der Bestrahlungsfelder und der Rasterpunkte

Im Folgenden wird nun beschrieben, wie die Bestrahlungsfelder bzw. die Rasterpunkte bestimmt werden, um dann hierauf die Bestrahlungsplanung durchzuführen, die auf einem neuen Abbildungsdatensatz beruht.

Nachdem ein neuer CT-Datensatz aufgezeichnet wurde und die Kontur und das Isozentrum des Zielvolumens bestimmt wurden, werden die Rasterpunkte wie folgt beschrieben verteilt.

Zunächst wird in Strahlrichtung die wasseräquivalente Pfadlänge (engl.:" water-equivalent path length", WEPL) für jedes Voxel des CT-Datensatzes berechnet. Hierzu kann das in M Krämer et al, "Treatment planning for heavy-ion radiotherapy: physical beam model and dose optimization", Phys. Med. Biol. 45 (2000) 3299-3317 beschriebene Verfahren eingesetzt werden, z.B. unter Verwendung der offenbarten Gleichung (7).

Anschließend werden die Energieschichten für die Planadaptation unter Berücksichtigung der WEPL des Zielvolumens festgelegt. Hierzu können im Wesentlichen die gleichen Energieabstände verwendet werden, wie sie auch für den oder die Referenzpläne festgelegt worden sind.

Anschließend wird ein neues Raster erzeugt, das das Isozentrum des Zielvolumens zum Ursprung hat. Auch hier können im Wesentlichen die gleichen Raster-Abstände gewählt werden wie sie den oder die Referenzpläne gelten.

Nun werden Rasterpunkte über das neue Raster verteilt, um das Zielvolumen auf jeder Energieschicht mit Rasterpunkten zu überdecken. Dabei können im Wesentliche ähnliche oder gleiche Erweitungen der Kontur des Zielvolumens verwendet werden wie sie auch in dem oder den Referenzplänen verwendet wurden.

### Adaptation der Partikelzahlen

Für die neuen Rasterpunkte können nun die Partikelzahlen bestimmt werden, indem die zuvor generierte DMC verwendet wird. Zunächst werden die neuen Rasterpunkte senkrecht zur Strahlrichtung projiziert.

Für jeden projizierten Rasterpunkt werden entlang der zugeordneten Strahllinie die Partikelzahlen für die Rasterpunkte entlang dieser Strahllinie bestimmt. Startpunkt ist hierbei diejenige Energieschicht, aus der der Strahl wieder aus dem Zielvolumen austritt. Der Reihe der vor diesem Startpunkt liegenden Rasterpunkte werden nun die Partikelzahlen der DMC zugeordnet. Zuvor wird die normierte DMC noch mit dem Energieniveau der distalsten Energieschicht, die im neuen Plan vorkommt, multipliziert. Auf diese Weise wird die zuvor durchgeführte Normierung rückgängig gemacht und Unterschiede hinsichtlich der für das Zielvolumen notwendigen Eindringtiefe zwischen Referenzplan und aktuellem Plan werden ausgeglichen.

Falls die Anzahl der von der Strahllinie durchdrungenen Isoenergieschichten größer ist als die Länge der DMC, können den überstehenden Rasterpunkten die letzte Partikelzahl der DMC zugeordnet werden. Dies ist akzeptabel, da der Schwanz einer DMC weitgehend flach ist.

### Ergebnisse

Im Folgenden werden die mit dem obigen Verfahren adaptierten Bestrahlungspläne untersucht. Basierend auf der Ähnlichkeit der DMCs für verschiedene Pläne der Prostata-BeispielPatienten und der Phantome für beide Felder wurden folgende Analysen durchgeführt, sowohl bei physikalisch optimierten als auch bei biologisch optimierten Plänen:
- Adaptation eines Referenzplans auf einen weiteren CT-Datensatz des gleichen Patienten (Intra-Patienten-Adaptation)
- Adaptation eines Patientenplans auf einen anderen Patienten (Inter-Patienten-Adaptation)
- Adaptation eines Phantomplans auf einen Patienten (Phantom-Patienten-Adaptation)
- Adaptation einer aus einem Feld abgeleiteten DMC auf beide Felder des adaptierten Plans (Inter-Feld-Adaptation)

### Intra-Patienten-Adaptation

Die Intra-Patienten-Adaptation wurde am Beispiel eines Prostata-Patienten für physikalische Bestrahlungspläne getestet.

Dabei wurde ein CT-Datensatz aus einer Serie von CT-Datensätzen des Patienten zufällig als Referenzdatensatz ausgewählt.

Die DMC wurde aus dem zugeordneten physikalischen Referenz-Bestrahlungsplan ermittelt. Adaptierte Bestrahlungspläne wurden mit dem oben beschriebenen Verfahren für die restlichen CT-Datensätze erzeugt.

Bei der Intra-Patienten-Adaptation wurde auf eine Normierung der DMC, bei der durch das distalste Energieniveau dividiert wurde, verzichtet, da für den gleichen Patienten etwaige Reichweitenunterschiede in Strahlrichtung so gering sind, dass der Einfluss auf die Partikelzahlen vernachlässigbar gering ist.

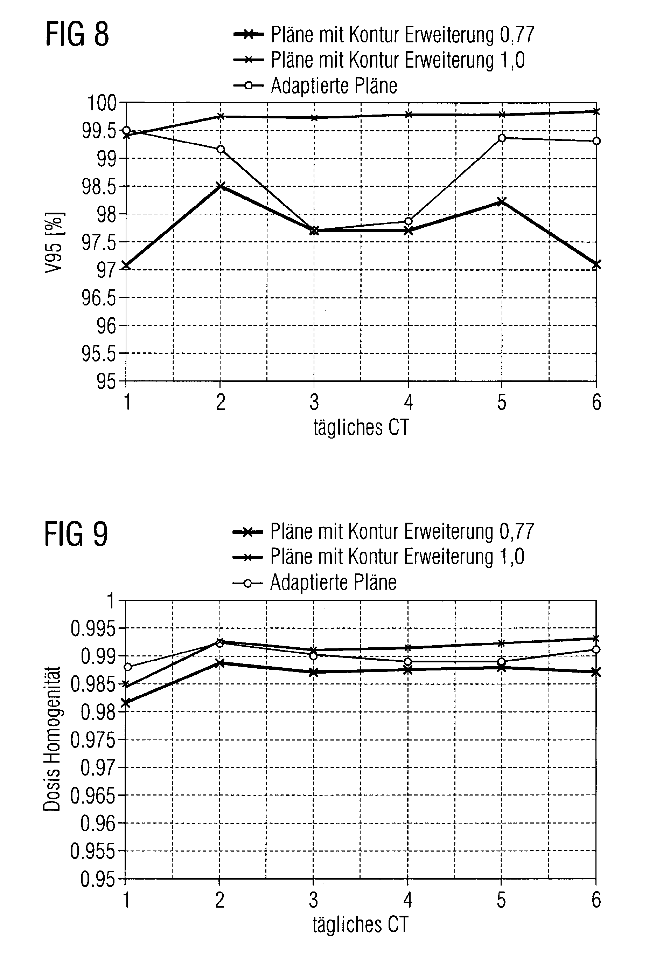

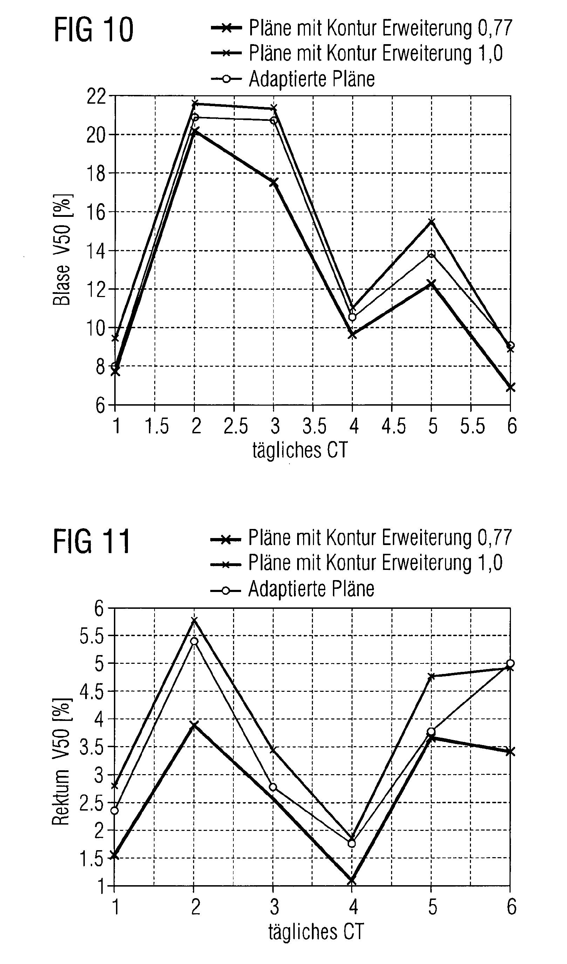

Es wurden die V95 des Zielvolumens (Fig. 8), die Homogenität innerhalb des Zielvolumen (engl.: "dose homogeneity"; Fig. 9), die V50 für Risikoorgane (im Falle der Prostata also die Blase (engl.: "bladder") und das Rektum (engl.: "rectum"); Fig. 10 und Fig. 11) für die adaptierten Pläne berechnet, die auf die jeweiligen CT-Datensätze angewendet werden, und mit den entsprechenden Größen derjenigen Plänen verglichen, die mit herkömmlichen Verfahren für die jeweiligen CT-Datensätze berechnet wurden.

In Fig. 8 bis Fig. 11 werden Pläne zu unterschiedlichen CT-Datensätzen (engl.: "daily CT"), die für eine Bestrahlungspläne mit einer Konturerweiterung (engl.: "plans with contour extension") von 0.77 und 1.0 mit einer herkömmlichen Optimierung berechet wurden, mit den adaptierten Plänen nach dem offenbarten Verfahren verglichen.

Es konnte eine zufriedenstellende Dosisabdeckung und Homogenität für das Zielvolumen, sowie eine vergleichbare Dosisbelastung für Risikoorgane erreicht werden.

### Inter-Patienten-Adaptation

Die Inter-Patienten-Adaptation wurde analog zur Intra-Patienten-Adaptation anhand von biologischen Plänen getestet.

Hierzu wurden biologische Plane für einen Beispielpatienten (Patient 2) gemäß dem oben erläuterten Ablauf für verschiedene CT-Datensätze adaptiert, indem die DMC verwendet wurde, die aus einem Plan eines anderen Beispielpatienten (Patient 3) ermittelt wurde.

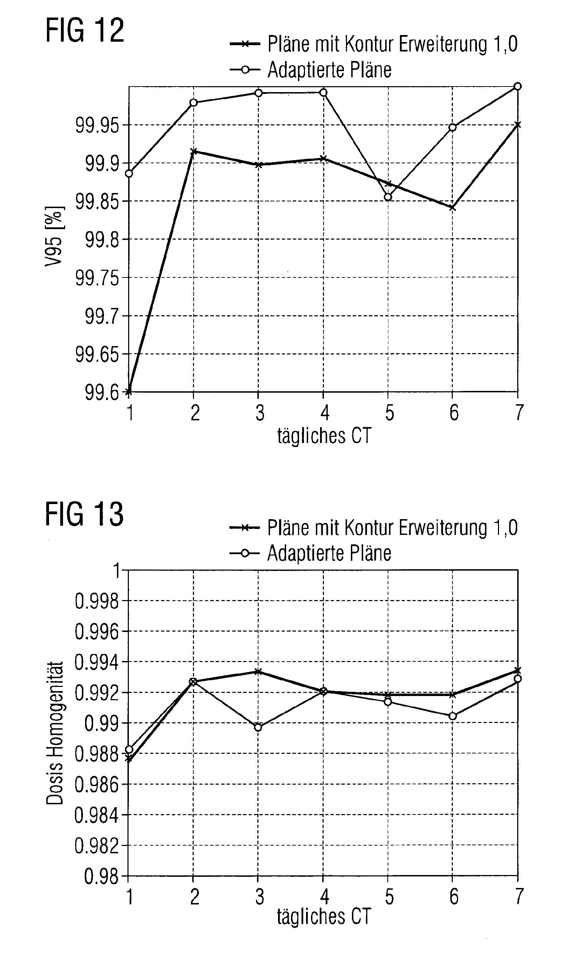

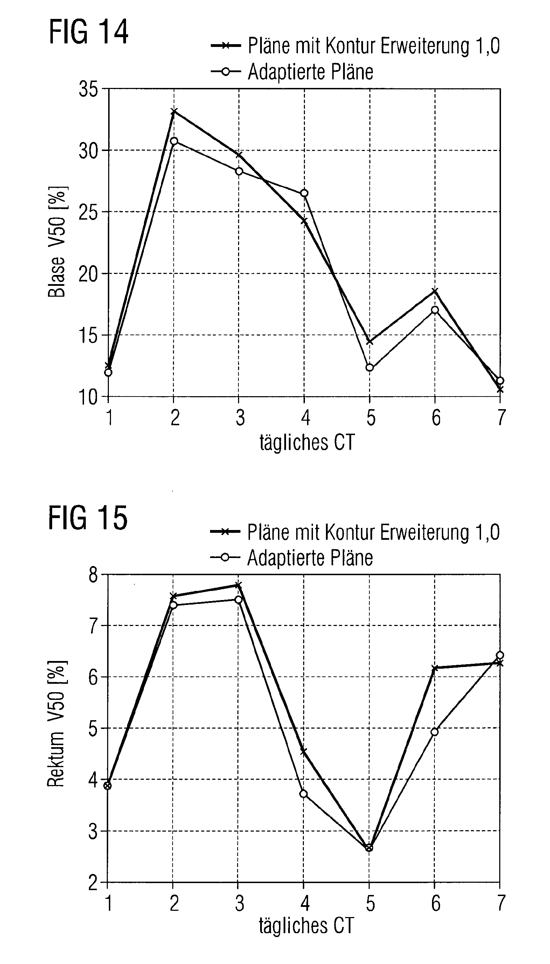

Wie in Fig. 12 bis Fig. 15 gezeigt, ist auch hier die Dosisüberdeckung die Homogenität sowie die Belastung der Risikoorgane zufriedenstellend.

### Phantom-Patienten-Adaptation und Inter-Feld-Adaptation

Diese beiden Planadaptationen wurden ebenfalls anhand von biologischen Plänen getestet.

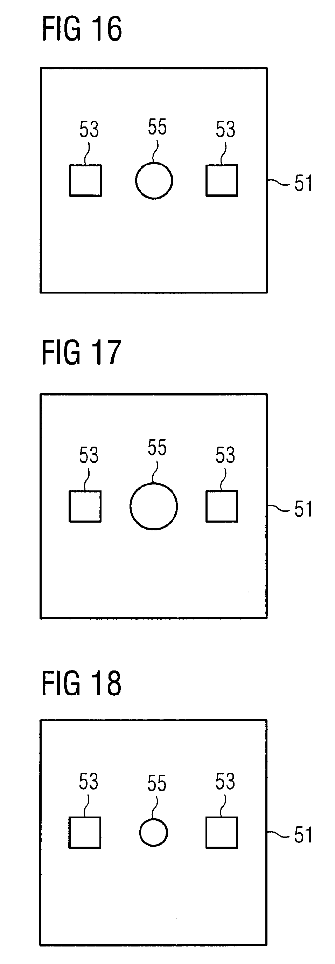

Für die Phantom-Patienten-Adaptation wurden drei Prostatakarzinom-Phantome mit unterschiedlichen Zielvolumina bzw. deren virtuelle CT-Datensätze mithilfe von MATLAB erzeugt. Fig. 16 bis Fig. 18 zeigt den Aufbau des Phantoms mit Körper 51, knöcherner Struktur 53 und Zielvolumen 55. Zu sehen ist die unterschiedliche Größe des Targets.

Eine mittlere DMC wurde berechnet, wobei für die Mittelung die normierten DMC der biologischen Pläne der drei Phantome zugrunde gelegen haben. Anhand dieser mittleren DMC wurde der Patientenplan auf die verschiedenen CT-Datensätze adaptiert. Dabei wurde die DMC eines Feldes (des +90°-Feldes) auf beide Felder im neuen Plan adaptiert, um die Inter-Feld-Adaptation zu testen.

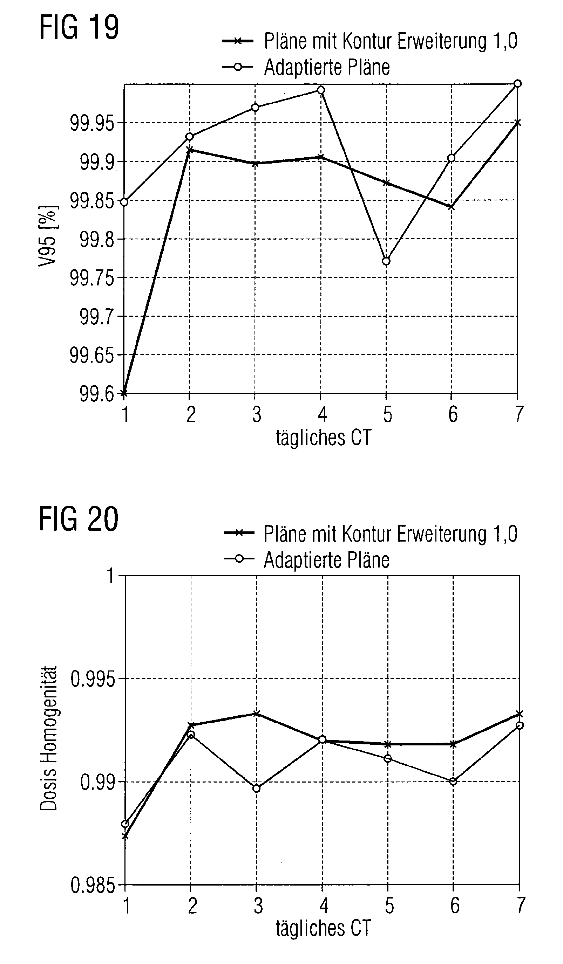

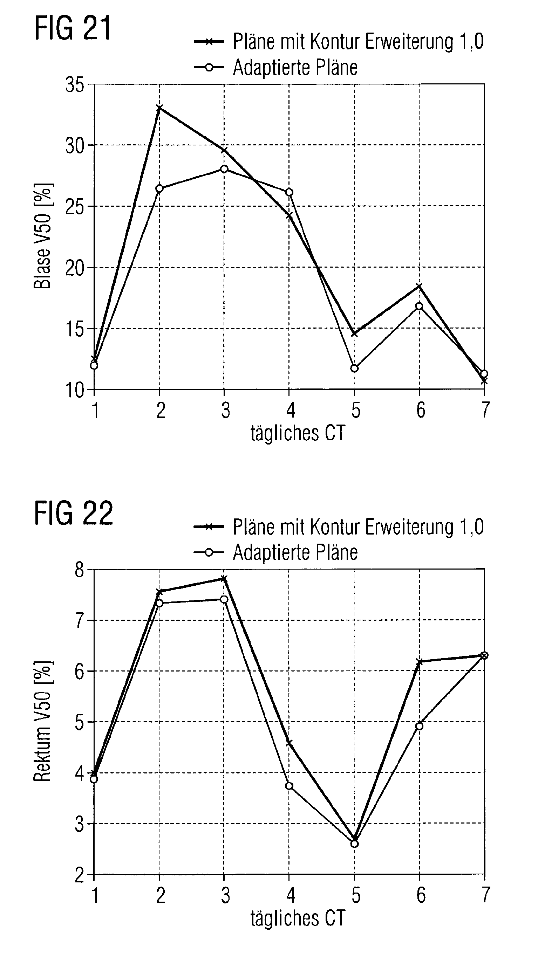

Fig. 19 bis Fig. 22 zeigt die Ergebnisse der Analyse analog zu Fig. 8 bis Fig. 11 bzw. Fig. 12 bis Fig. 15.

Auch hier ist die Dosisüberdeckung, die Homogenität sowie die Belastung der Risikoorgane zufriedenstellend, verglichen mit den für die CT-Datensätze herkömmlich berechneten Plänen. Grenzen des Verfahren und Lösung

Das Verfahren eignet sich am besten für konvexe Zielvolumina mit konsekutiven Zielpunkten entlang der Strahlrichtung. Andernfalls kann der Fall eintreten, dass die Dosisverteilung und die Schonung von Risikoorganen beeinträchtigt sind.

Dennoch ist es denkbar, für solche Fälle ein sogenanntes Field-Patching durchzuführen, bei dem ein konkaves Zielvolumen in mehrere konvexe Teile aufgeteilt wird, um den Fall eines konkaven Zielvolumens bei der Planadaptation zu umgehen.

Das erfindungsgemäße Adaptationsverfahren zur Planung bzw. zum Re-Planning basierend auf einer DMC zur Bestimmung der Partikelzahlen für Rasterpunkte erlaubt es, einen Plan sehr schnell zu berechnen im Vergleich zu den aufwändigen iterativen Dosisoptimierung insbesondere für biologische Pläne.

Im Test mit Phantom-Datensätzen und Datensätzen verschiedener Prostata-Patienten können bei einer Intra-Patienten-Adaptation, bei einer Inter-Patienten-Adaptation, bei einer Phantom-Patienten Adaptation und auch bei einer Inter-Feld-Adaptation eine exzellente Zielvolumenüberdeckung und Dosishomogenität, und gleichzeitig eine ähnliche Dosisbelastung für Risikoorgane erreicht werden.

Der Vergleich bezieht sich dabei auf den Vergleich von Plänen, die mit dem erfindungsgemäßen Verfahren bestimmt wurden, mit Plänen, die nach einem herkömmlichen Verfahren berechnet wurden.

Die Planberechnung kann z.B. bei Implementierung mithilfe des kommerziell verfügbaren MATLAB sehr schnell durchgeführt werden, üblicherweise in weniger als einer Minute, und hat daher das Potential, selbst zur online Adaptation zur Kompensation von inter-fraktionellen Patientenbewegungen verwendet zu werden.

Daneben kann das Verfahren aber auch zum schnellen Offline-Re-Planning als auch zur Neuplanung von Patientenbestrahlungen eingesetzt werden.

## Patentansprüche

1. Verfahren zur Erstellung eines Bestrahlungsplans für ein Zielvolumen, umfassend folgende Schritte:
- Bereitstellen einer Modulationskurve, welche die Modulation von Partikelzahlen bei Zielpunkten, welche im Zielvolumen in Strahlrichtung hintereinander liegen, charakterisiert,
- Ermitteln einer aktuellen Lage des Zielvolumens,
- Bestimmen einer Vielzahl von aktuellen Zielpunkten, welche das Zielvolumen in seiner aktuellen Lage überdeckenden,
- Zuordnen von Partikelzahlen zu den aktuellen Zielpunkten unter Verwendung der Modulationskurve.

2. Verfahren nach Anspruch 1,
wobei in einem Vorverfahren die Modulationskurve aus mindestens einem bestehenden Bestrahlungsplan, in welchem eine Zuordnung von Partikelzahlen zu Zielpunkten des Zielvolumens hinterlegt ist, ermittelt wird.

3. Verfahren nach Anspruch 2,
wobei das Zielvolumen, das in dem Vorverfahren zur Ermittlung der Modulationskurve verwendet wird, zu derselben Person gehört wie das Zielvolumen, für das die aktuelle Lage ermittelt wird, oder
wobei das Zielvolumen, das in dem Vorverfahren zur Ermittlung der Modulationskurve verwendet wird, zu einer anderen Person gehört als das Zielvolumen, für das die aktuelle Lage ermittelt wird, oder
wobei das Zielvolumen, das in dem Vorverfahren zur Ermittlung der Modulationskurve verwendet wird, zu einem Phantom gehört, welches das Zielvolumen nachbildet.

4. Verfahren nach Anspruch 2 oder 3,
wobei bei dem Vorverfahren die Modulationskurve ermittelt wird, indem Partikelzahlen mehrerer Zielpunktlinien gemittelt werden, wobei eine Zielpunktlinie durch in Strahlrichtung hintereinander liegende Zielpunkte gegeben ist.

5. Verfahren nach einem der obigen Ansprüche, wobei die Modulationskurve hinsichtlich des für die Bestrahlung des Zielvolumens notwendigen Energieniveaus des Partikelstrahls normiert ist.

6. Verfahren nach einem der obigen Ansprüche,
wobei beim Bestimmen der Vielzahl von Zielpunkten, die das Zielvolumen überdecken, ein Zielpunktabstand gewählt wird, der einem bereits bestehenden Referenzplan entspricht.

7. Verfahren nach einem der obigen Ansprüche,
wobei beim Zuordnen von Partikelzahlen zu den aktuellen Zielpunkten einer Zielpunktlinie, die aus aktuellen Zielpunkten gebildet ist, Partikelzahlen zugeordnet werden, die durch die Modulationskurve beschrieben sind.

8. Verfahren nach Anspruch 7,
wobei Partikelzahlen, die durch die Modulationskurve beschrieben sind, vor Zuordnung zu der Zielpunktlinie aus aktuellen Zielpunkten hinsichtlich des Energieniveaus, das für die Bestrahlung des Zielvolumens notwendig ist, angepasst werden.

9. Verfahren nach einem der obigen Ansprüche,
wobei das Verfahren zur Neuberechnung eines bereits berechneten Bestrahlungsplans mit optimierter physikalischer oder biologischer Dosisverteilung eingesetzt wird.

10. Verfahren nach einem der obigen Ansprüche,
wobei das Verfahren zur Übertragung eines bereits bestehenden Bestrahlungsplans auf einen neuen Bestrahlungsplan eingesetzt wird,
- wobei der bestehende Bestrahlungsplan und der neue Bestrahlungsplan dem gleichen Patienten zugeordnet sind, oder
- wobei der bestehende Bestrahlungsplan und der neue Bestrahlungsplan unterschiedlichen Patienten zugeordnet sind, oder
- wobei der bestehende Bestrahlungsplan einem Phantom und der neue Bestrahlungsplan einem Patienten zugeordnet ist.

11. Verfahren nach einem der obigen Ansprüche,
wobei das Verfahren zur Bestimmung von Partikelzahlen eines ersten Bestrahlungsfeldes durchgeführt wird, wobei die Modulationskurve aus einem zweiten Bestrahlungsfeld ermittelt wird, das im Vergleich zum ersten Bestrahlungsfeld einen unterschiedlichen Einstrahlwinkel aufweist.

12. Verfahren nach einem der obigen Ansprüche,
wobei mehrere Modulationskurven bereitgestellt werden, welche aus unterschiedlichen Bestrahlungsplänen, die sich hinsichtlich Zielorgan, Zieldosis, zu verwendende Teilchensorte und/oder Zielpunktabständen unterscheiden, erzeugt worden sind,
und bei der Zuordnung von Partikelzahlen zu den aktuellen Zielpunkten eine der Modulationskurven ausgewählt wird.

13. Bestrahlungsplanungsvorrichtung mit einer Rechnervorrichtung, umfassend:
eine Komponente zum Bereitstellen einer Modulationskurve, welche die Modulation von Partikelzahlen bei Zielpunkten, welche im Zielvolumen in Strahlrichtung hintereinander liegend, charakterisiert,
eine Komponente zum Ermitteln einer aktuellen Lage des Zielvolumens,
eine Komponenten zum Bestimmen einer Vielzahl von aktuellen Zielpunkten, welche das Zielvolumen in seiner aktuellen Lage überdeckenden,
eine Komponente zum Zuordnen von Partikelzahlen zu den aktuellen Zielpunkten unter Verwendung der Modulationskurve.

14. Bestrahlungsplanungsvorrichtung nach Anspruch 13, wobei die Rechnervorrichtung konfiguriert ist zur Durchführung eines Verfahrens nach einem der Ansprüche 1 bis 12.

## Claims

1. Method for producing an irradiation plan for a target volume, incorporating the following steps:
- preparation of a modulation curve which characterises the modulation, in the direction of the beam, of particle numbers at target points which lie one behind another in the target volume,
- determination of the current position of the target volume,
- definition of a plurality of current target points, which fully cover the target volume in its current position,
- assignment of particle numbers to the current target points, using the modulation curve.

2. Method according to claim 1,
in which, in a preliminary procedure, the modulation curve is determined from at least one existing irradiation plan in which is stored an assignment of particle numbers to target points in the target volume.

3. Method according to claim 2,
where the target volume, used in the preliminary procedure for the determination of the modulation curve, belongs to the same person as the target volume for which the current position is being determined, or
where the target volume, used in the preliminary procedure for the determination of the modulation curve, belongs to a different person than does the target volume for which the current position is being determined, or
where the target volume, used in the preliminary procedure for the determination of the modulation curve, belongs to a phantom which simulates the target volume.

4. Method according to claim 2 or 3,
in which the modulation curve is determined in the preliminary procedure by averaging particle numbers from several target point lines, where a target point line is defined by target points lying one behind another in the direction of the beam.

5. Method according to one of the above claims, in which the modulation curve is normalised in terms of the energy level of the particle beam which is required for irradiation on the target volume.

6. Method according to one of the above claims, in which in defining the plurality of target points which fully cover the target volume, a target point spacing is selected which corresponds to a reference plan which already exists.

7. Method according to one of the above claims, in which in assigning particle numbers to the current target points on a target point line, formed by current target points, particle numbers defined by the modulation curve are assigned.

8. Method according to claim 7, in which before they are assigned to the target point line of current target points, the particle numbers which are specified by the modulation curve are adapted in terms of the energy level which is required for the irradiation on the target volume.

9. Method according to one of the above claims, in which the method is used for recalculation of an irradiation plan which has already been calculated with optimised physical or biological dose distribution.

10. Method according to one of the above claims, in which the method is used for transferring an irradiation plan which already exists over to a new irradiation plan,
- where the existing irradiation plan and the new irradiation plan are assigned to the same patient, or
- where the existing irradiation plan and the new irradiation plan are assigned to different patients, or
- where the existing irradiation plan is assigned to a phantom and the new irradiation plan to a patient.

11. Method according to one of the above claims, in which the method for determining particle numbers is carried out for a first irradiation field, whereby the modulation curve is determined from a second irradiation field which, by comparison with the first irradiation field, has a different angle of incidence.

12. Method according to one of the above claims, in which several modulation curves are prepared, these having been
produced from different irradiation plans which differ in respect of the target organ, target dose, the type of particle to be used and/or target point spacing,
and one of the modulation curves is selected in making the assignment of particle numbers to the current target points.

13. Irradiation planning apparatus with a computing facility incorporating:
a component for preparing a modulation curve, which characterises the modulation of particle numbers at target points which, in the direction of the beam, lie one behind another in the target volume,
a component for determining the current position of the target volume,
a component for defining a plurality of current target points which fully cover the target volume in its current position,
a component for assigning particle numbers to the current target points, making use of the modulation curve.

14. Irradiation planning apparatus according to claim 13, in which the computing facility is configured for the purpose of carrying out a method according to one of the claims 1 to 12.

## Revendications

1. Procédé d'établissement d'un plan d'exposition d'un volume cible, comprenant les stades suivants :
- mise à disposition d'une courbe de modulation qui caractérise la modulation de nombre de particules en des points cibles qui se succèdent dans la direction du faisceau dans le volume cible,
- détermination d'une position présente du volume cible,
- définition d'une pluralité de points cibles présents qui recouvrent le volume cible en sa position présente,
- affectation de nombres de particules aux points cibles présents en utilisant la courbe de modulation.

2. Procédé suivant la revendication 1,
dans lequel, dans un procédé préalable, on détermine la courbe de modulation à partir d'au moins un plan d'exposition existant dans lequel est mémorisée une affectation de nombres de particules à des points cibles du volume cible.

3. Procédé suivant la revendication 2,
dans lequel le volume cible, qui est utilisé dans le procédé préalable de détermination de la courbe de modulation, appartient à la même personne que le volume cible dont on détermine la position présente, ou
dans lequel le volume cible, qui est utilisé dans le procédé préalable de détermination de la courbe de modulation, appartient à une autre personne que le volume cible dont on détermine la position présente, ou
dans lequel le volume cible, qui est utilisé dans le procédé préalable de détermination de la courbe de modulation, appartient à un fantôme qui reproduit le volume cible.

4. Procédé suivant la revendication 2 ou 3,
dans lequel, dans le procédé préalable, on détermine la courbe de modulation en faisant la moyenne de nombres de particules de plusieurs lignes de points cibles, une ligne de points cibles étant donnée par des points cibles se trouvant les uns derrière les autres dans la direction du faisceau.

5. Procédé suivant l'une des revendications précédentes,
dans lequel la courbe de modulation est normée du point de vue du niveau d'énergie du faisceau de particules nécessaire pour l'exposition du volume cible.

6. Procédé suivant l'une des revendications précédentes,
dans lequel, dans la détermination de la pluralité de points cibles qui recouvrent le volume cible, on choisit une distance entre les points cibles qui correspond à un plan de référence déjà existant.

7. Procédé suivant l'une des revendications précédentes,
dans lequel, dans l'affectation de nombres de particules aux points cibles présents d'une ligne de points cibles, qui est formée de points cibles présents, on affecte des nombres de particules qui sont décrits par la courbe de modulation.

8. Procédé suivant la revendication 7,
dans lequel on adapte des nombres de particules qui sont décrits par la courbe de modulation avant l'affectation à la ligne de points cibles à partir de points cibles présents, en ce qui concerne le niveau d'énergie qui est nécessaire pour l'exposition du volume cible.

9. Procédé suivant l'une des revendications précédentes, dans lequel on utilise le procédé pour recalculer un plan d'exposition déjà calculé avec une répartition de dose physique ou biologique optimisée.

10. Procédé suivant l'une des revendications précédentes, dans lequel on utilise le procédé pour transformer un plan d'exposition déjà existant en un plan d'exposition nouveau,
- dans lequel le plan d'exposition existant et le plan d'exposition nouveau sont associés au même patient, ou
- dans lequel le plan d'exposition existant et le plan d'exposition nouveau sont associés à des patients différents, ou
- dans lequel le plan d'exposition existant est associé à un fantôme et le plan d'exposition nouveau à un patient.

11. Procédé suivant l'une des revendications précédentes, dans lequel on effectue le procédé pour la détermination de nombres de particules d'un premier champ d'exposition, la courbe de modulation étant déterminée à partir d'un deuxième champ d'exposition qui, par rapport au premier champ d'exposition, a un angle d'arrivée différent.

12. Procédé suivant l'une des revendications précédentes, dans lequel on met à disposition plusieurs courbes de
modulation qui ont été produites à partir de plans d'exposition différents, lesquels se distinguent en ce qui concerne l'organe cible, la dose cible, le type de particules à utiliser et/ou les distances entre points cibles,
et, lors de l'affectation de nombres de particules aux points cibles présents, on choisit l'une des courbes de modulation.

13. Dispositif de planification d'exposition ayant un dispositif d'ordinateurs comprenant :
un composant de mise à disposition d'une courbe de modulation, qui caractérise la modulation de nombres de particules pour des points cibles qui sont les uns derrière les autres dans la direction du faisceau dans le volume cible,
un composant de détermination d'une position présente du volume cible,
un composant de définition d'une pluralité de points cibles présents qui recouvrent le volume cible dans sa position présente,
un composant d'affectation de nombres de particules aux points cibles présents en utilisant la courbe de modulation.

14. Dispositif de planification d'exposition suivant la revendication 13, dans lequel le dispositif d'ordinateur est configuré pour effectuer un procédé suivant l'une des revendications 1 à 12.
